# EUROPEAN PATENT APPLICATION

(11) **EP 0 752 254 A1**
(43) Date of publication of application: **08.01.1997**
(21) Application number: 95905765.4
(22) Date of filing: 12.01.1995
(51) Int. Cl.: A61N 5/06

(54) **INFRARED TREATMENT DEVICE FOR PHYSICAL EXAMINATION AND TREATMENT**

(71) Applicant: TOKYO IKEN CO., LTD., Tokyo 206 (JP)
(72) Inventor: KONDO, Hiroaki, Kawasaki-shi, Kanagawa 215 (JP)
(74) Representative: W.P. Thompson & Co.
(86) International application number: JP9500028
(87) International publication number: WO9621490

(57) **Abstract**

An infrared treatment device for physical examination and treatment wherein a halogen lamp (1) generates a light containing therein infrared rays, and a flexible optical fiber light guide (3) serves to guide the light containing therein infrared rays to the affected part. A color glass filter (6) removes a light of a predetermined wavelength range from a light which includes infrared rays outgoing from a tip end surface of the optical fiber light guide (3), a group of condensing lens (7) condenses a light which includes infrared rays having passed through the color glass filter (6), and a linear polarization plate (8) subjects a light, which is condensed by the group of condensing lens (7) and includes infrared rays, to linear polarization to apply the same onto the affected part. By this means, the affected part is treated by a light which includes infrared rays.

## Description

### Technical Field

The present invention relates to an infrared therapeutic instrument for physical therapy and, more particularly, to an infrared therapeutic instrument for physical therapy for directing infrared radiation to a diseased part to treat the diseased part by a thermal effect or the like.

### Background Art

This kind of conventional infrared therapeutic instrument for physical therapy is a simple one generally designed to reflect infrared radiation containing light generated from an infrared lamp or the like on a reflecting mirror and direct the reflected light to a diseased part.

In the conventional infrared therapeutic instrument for physical therapy mentioned above, the infrared radiation containing light generated from the infrared lamp or the like is reflected on the reflecting mirror to be widely directed to the diseased part. Accordingly, a small diseased part cannot be irradiated with infrared radiation having a sufficient energy density. As the range of irradiation is wide, another part not to be irradiated is unnecessarily irradiated with the infrared radiation containing light.

It is accordingly an object of the present invention to provide an infrared therapeutic instrument for physical therapy employing an optical fiber lightguide having a flexibility for guiding to a diseased part infrared radiation containing light generated from a halogen lamp.

### Disclosure of Invention

The infrared therapeutic instrument for physical therapy of the present invention comprises a halogen lamp for generating infrared radiation containing light; an optical fiber lightguide having a flexibility for guiding the infrared radiation containing light to a diseased part; a color glass filter for cutting off light having a waveband in a predetermined range from the infrared radiation containing light emerging from a front end surface of the optical fiber lightguide; condenser lenses for condensing the infrared radiation containing light passed through the color glass filter; and a linearly polarizing plate for linearly polarizing the infrared radiation containing light condensed by the condenser lenses.

According to the present invention, the infrared radiation containing light generated from the halogen lamp is guided to the diseased part by using the optical fiber lightguide having a flexibility. Therefore, even a small diseased part can be irradiated with the infrared radiation having a sufficient energy density.

Furthermore, as the optical fiber lightguide has a flexibility, the instrument is easy to handle and convenient to use. In addition, the infrared radiation can be directed to a diseased part at any position.

Accordingly, the infrared therapeutic instrument for physical therapy of the present invention can spot-direct the infrared radiation with a high energy density to a diseased part, and therefore has a curative effect on the following diseases.
(1) Remission of pains
   Subacute or chronic pains of muscles, joints, etc. and neuralgia
   ① Neck ... pain of the cervical spine, whiplash syndrome, crick of the neck in the sleep
   ② Shoulder and Back ... stiff and painful shoulder, back pain, distortion of the shoulder joint
   ③ Loins ... strained back, sciatica, pain by hernia of intervertebral discs
   ④ Arm ... elbow arthralgia, tennis elbow, snapping thumb pain
   ⑤ Leg ... knee arthralgia
(2) Sedation of inflammatory pains, other pains by trauma (after an acute period), distortion, fracture, inflammation of the tendon, inflammation of the tendon sheath, strained back, injury of the deep muscle and the ligament, and spasm of the muscle
(3) Arthritis and rheumatism
   Rheumatic arthritis, arthritis deformans (except acute and subacute ones)
(4) Skin diseases
   Chronic dermatitis, acne vulgaris, decubitus

### Brief Description of Drawings

Fig. 1 is a partially cutaway, side view showing an essential part of an infrared therapeutic instrument for physical therapy according to a preferred embodiment of the present invention;
Fig. 2(a) is a partially sectional, exploded, enlarged side view of a front end portion of a probe shown in Fig. 1;
Fig. 2(b) is an assembled, enlarged side view of the front end portion of the probe;
Fig. 2(c) is a completed, enlarged side view of the front end portion of the probe;
Fig. 3 is a side view of an essential part illustrating the divergence of rays of light emerging from the front end surface of the probe in the condition that a lens barrel shown in Fig. 1 is removed;
Fig. 4(a) is a perspective view illustrating rays of light spot-directed to a diseased part in the condition that the lens barrel is mounted to the probe;
Fig. 4(b) is a perspective view illustrating rays of light widely directed to a diseased part in the condition that the lens barrel is removed from the probe; and
Fig. 5 is an illustration of a wavelength distribution in the infrared therapeutic instrument for physical therapy according to the preferred embodiment.

### Best Mode for Carrying Out the Invention

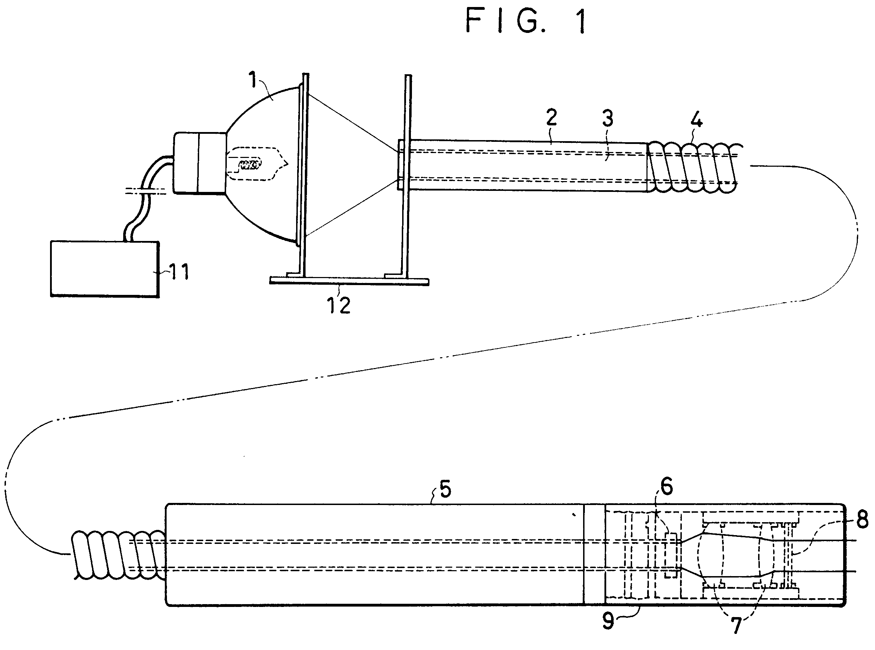

Fig. 1 is a partially cutaway, side view showing an essential part of an infrared therapeutic instrument for physical therapy according to a preferred embodiment of the present invention. The infrared therapeutic instrument for physical therapy according to the preferred embodiment is mainly composed of a halogen lamp 1, a protective pipe 2, an optical fiber lightguide 3, a vinyl chloride tube 4, a probe 5, a color glass filter 6, condenser lenses 7, a linearly polarizing plate 8, and a lens barrel 9. Reference numeral 11 denotes a variable-output power circuit for supplying electric power to the halogen lamp 1, and reference numeral 12 denotes a support member for fixing the halogen lamp 1 and the protective pipe 2 as a unit.

The inner surface of the halogen lamp 1 is mirror-coated with metal by vapor deposition, so as to allow visible light and infrared radiation diffused from a light source to efficiently enter the base end surface of the optical fiber lightguide 3.

The protective pipe 2 is a pipe formed of aluminum or the like, for protecting the base end portion of the optical fiber lightguide 3.

The optical fiber lightguide 3 is formed by bundling 19,000 optical fibers each having a diameter of 50 µm with a quartz multi-component structure and being composed of a core and a clad different from each other in refractive index. The optical fiber lightguide 3 does not transmit infrared radiation having a long waveband of 1.6 µm or more.

The vinyl chloride tube 4 is provided to insert the optical fiber lightguide 3 therethrough and protect it from surroundings so as to prevent excess bend of the optical fiber lightguide 3.

The probe 5 is fixed to the front end portion of the optical fiber lightguide 3. The probe 5 is adapted to be gripped by a hand or arms for manipulation.

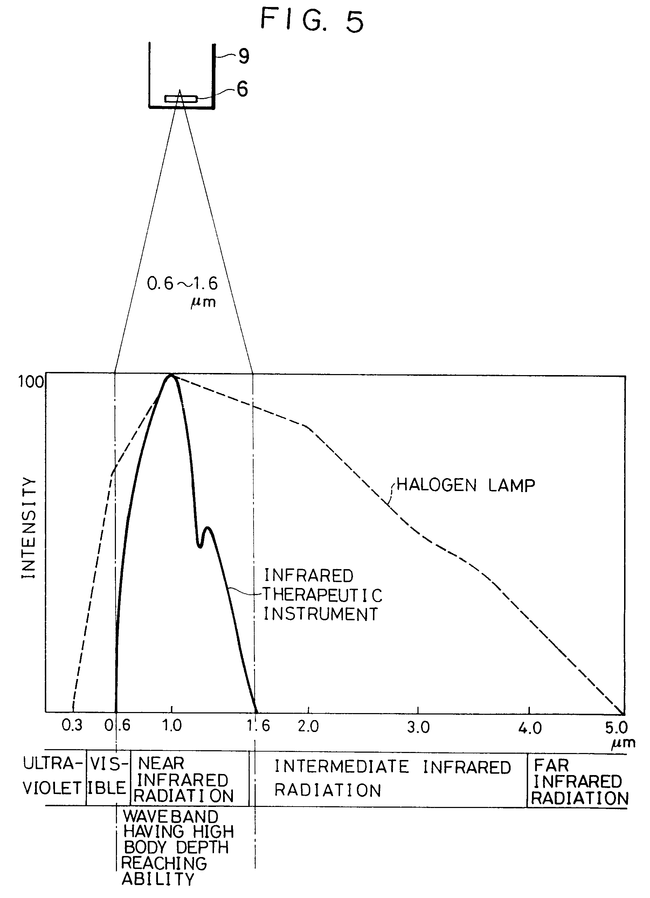

The color glass filter 6 is formed from a color glass plate, and it serves to absorb light having a short waveband of 0.6 µm or less, which is low in body depth reaching ability, since the light to be emitted from the halogen lamp 1 ranges from ultraviolet radiation having a wavelength of 0.3 µm to infrared radiation having a wavelength of 5.0 µm (see Fig. 5).

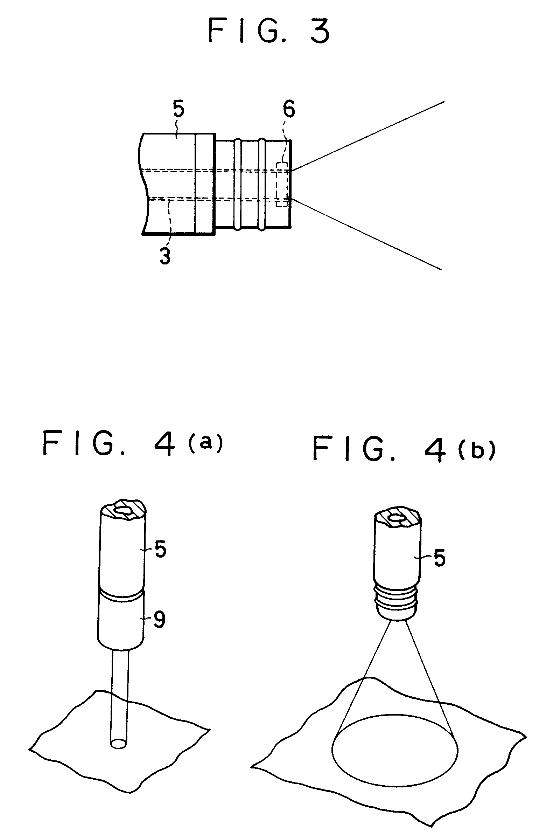

The condenser lenses 7 are lenses for condensing light diverging from the front end surface of the optical fiber lightguide 3 to obtain sectionally circular rays of light approximate to parallel rays, because the light from the front end surface of the optical fiber lightguide 3 diverges at an angle of about 30° (see Fig. 3).

The linearly polarizing plate 8 is formed by dyeing a polyvinyl alcohol film with an iodine liquid and sandwiching this film between optical glass plates. The linearly polarizing plate 8 serves to linearize a plane of polarization of light having a waveband of about 0.6 to 1.6 µm. It is known that the infrared radiation thus linearly polarized has a remarkable effect on activation of biopolymer, improvement in microcirculation, etc. to improve a therapeutic effect.

The lens barrel 9 is removably mounted at the front end portion of the probe 5. When the lens barrel 9 is removed from the probe 5, the diverging rays of light are allowed to emerge from the front end surface of the optical fiber lightguide 3 at a divergence angle of about 30° (see Fig. 4(b)), whereas when the lens barrel 9 is mounted to the probe 5, the sectionally circular rays of light approximate to parallel rays are allowed to emerge from the front end of the lens barrel 9 (see Fig. 4(a)).

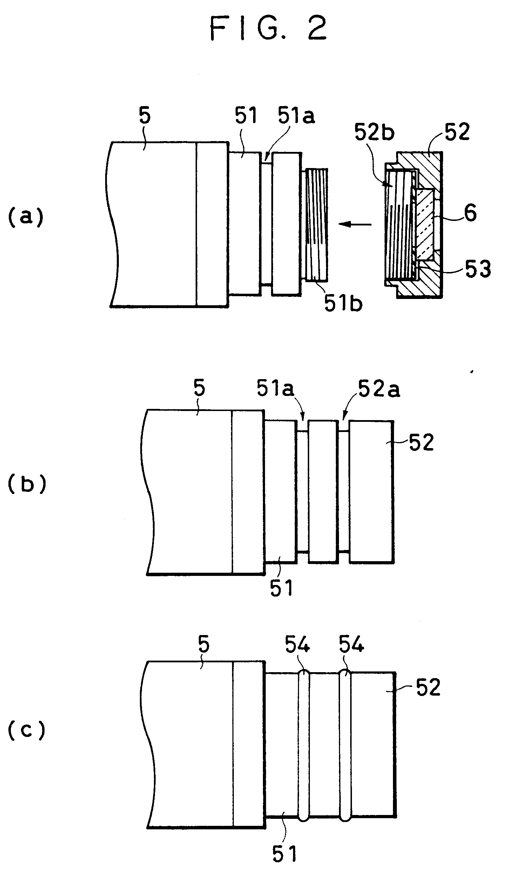

Figs. 2(a), 2(b), and 2(c) are a partially sectional, exploded, enlarged side view, an assembled, enlarged side view, and a completed, enlarged side view of the front end portion of the probe 5, respectively. As shown in Fig. 2(a), the front end portion of the probe 5 is provided with a step portion 51 reduced in diameter. The step portion 51 is formed at its intermediate position with a groove 51a, and is further formed at the front end with a male screw 51b. The male screw 51b is adapted to engage with a female screw 52b formed on the inner surface of a front cap 52 having the same diameter as that of the step portion 51. The front cap 52 is formed with a central through hole. The color filter plate 6 and a packing member 53 formed of silicone resin are mounted inside the front cap 52 so as to be aligned with the central through hole of the front cap 52. In the condition where the female screw 52b is threadedly engaged with the male screw 51b as shown in Fig. 2(b), the front cap 52 is integrated with the step portion 51, and the packing member 53 is pressed on the front end surface of the step portion 51 to thereby fix the color glass filter 6 within the front cap 52. Further, in this engaged condition of the front cap 52 and the step portion 51, a groove 52a similar to the groove 51a is formed between the step portion 51 and the front cap 52. As shown in Fig. 2(c), O-rings 54 formed of silicone resin are fitted with these grooves 51a and 52a. Accordingly, when the lens barrel 9 is engaged with the probe 5, the lens barrel 9 is air-tightly and water-tightly fixed to the probe 5 by a frictional force of the O-rings 54.

The operation of the infrared therapeutic instrument for physical therapy according to the preferred embodiment mentioned above will now be described.

The infrared radiation containing light emitted from the halogen lamp 1 is allowed to enter the base end surface of the optical fiber lightguide 3.

The optical fiber lightguide 3 cuts off the infrared radiation having a long waveband of 1.6 µm or more, but allows to transmit the light having another waveband. The transmitted light passes through the core of each optical fiber as being reflected by the clad to reach the front end surface of the optical fiber lightguide 3, and is then allowed to emerge from the front end surface of the optical fiber lightguide 3.

The infrared radiation containing light emerging from the front end surface of the optical fiber lightguide 3 is filtered by the color glass filter 6 to allow pass of only the light having a waveband of about 0.6 to 1.6 µm, which is high in body depth reaching ability. Then, the light passing through the color glass filter 6 is allowed to emerge from the front end surface of the probe 5.

The light emerging from the front end surface of the probe 5 is condensed by the condenser lenses 7, and is then linearly polarized by the linearly polarizing plate 8. Then, the light thus linearly polarized is directed to a diseased part.

The optical fiber lightguide 3 has a flexibility and can be curved. Therefore, the infrared radiation can be concentrated and spot-directed to a diseased part at any position with a high energy density as shown in Fig. 4(a) by operating the probe 5.

In the condition that the lens barrel 9 is not mounted to the probe 5, the infrared radiation may be widely directed to a diseased part as shown in Fig. 4(b), because the light emerging from the front end surface of the probe 5 diverges at an angle of about 30°.

As shown in Fig. 5, the light emerging from the front end surface of the lens barrel 9 contains about 5% of red visible light and about 95% of invisible near infrared radiation capable of reaching the depth of a living body and being absorbed to give a thermal effect. Further, the light emerging from the front end surface of the lens barrel 9 has a peak of intensity at a wavelength near 1.0 µm, and the light having a waveband of about 0.6 to 1.6 µm has a high body depth reaching ability to promote warming and activation of the depth of a living body.

As the infrared therapeutic instrument for physical therapy according to the preferred embodiment employs the halogen lamp 1, the output from the halogen lamp 1 can be easily varied by the variable-output power circuit 11. For example, the output from the halogen lamp 1 can be continuously varied in the range of 0.0 to 1.8 W. When the infrared radiation is directed to a diseased part in the condition that the probe 5 or the lens barrel 9 is in contact with the diseased part, the output from the halogen lamp 1 may be adjusted to a low output, whereas when the infrared radiation is directed in the condition that the probe 5 or the lens barrel 9 is kept away from the diseased part, the output may be adjusted to a high output.

### FIG. 5

1: HALOGEN LAMP
2: INFRARED THERAPEUTIC INSTRUMENT OF THE INVENTION
3: INTENSITY
4: ULTRAVIOLET RADIATION
5: VISIBLE LIGHT
6: NEAR INFRARED RADIATION
7: INTERMEDIATE INFRARED RADIATION
8: FAR INFRARED RADIATION
9: WAVEBAND HAVING HIGH BODY DEPTH REACHING ABILITY

## Claims

1. An infrared therapeutic instrument for physical therapy comprising:
a halogen lamp (1) for generating infrared radiation containing light;
an optical fiber lightguide (3) having a flexibility, for guiding said infrared radiation containing light to a diseased part;
a color glass filter (6) for cutting off light having a waveband in a predetermined range from said infrared radiation containing light emerging from a front end surface of said optical fiber lightguide (3);
condenser lenses (7) for condensing said infrared radiation containing light passed through said color glass filter (6); and
a linearly polarizing plate (8) for linearly polarizing said infrared radiation containing light condensed by said condenser lenses (7).
